# EUROPEAN PATENT APPLICATION

(11) **EP 3 858 363 A1**
(43) Date of publication of application: **04.08.2021**
(21) Application number: 20305072.9
(22) Date of filing: 28.01.2020
(51) Int. Cl.: A61K 35/00, A61P 1/00, A61P 11/00, A61P 31/04

(54) **COMPOSITION FOR TREATING INTESTINAL OR PULMONARY DISEASES**

(71) Applicant: Institut national de recherche pour l'agriculture, l'alimentation et l'environnement, 75007 Paris (FR); CENTRE NATIONAL DE LA RECHERCHE SCIENTIFIQUE - CNRS, 75016 Paris 16 (FR); INSTITUT NATIONAL DE LA SANTE ET DE LA RECHERCHE MEDICALE - INSERM, 75013 Paris (FR); Université de Lille, 59000 Lille (FR); Institut Pasteur De Lille, 59000 Lille (FR)
(72) Inventor: THOMAS, Muriel, 91430 IGNY (FR); TROTTEIN, François, 59200 TOURCOING (FR); VERSTRAETEN, Sophie, 91300 MASSY (FR); SENCIO, Valentin, 59200 Lille (FR)
(74) Representative: Nony

(57) **Abstract**

This disclosure relates to a composition for use in a method for treating and/or preventing intestinal and/or pulmonary disease in a subject in need thereof, wherein the composition comprises, in a physiologically acceptable condition, an effective amount of at least one acetate-overproducing bacteria hypersensitive to oxygen.

## Description

### FIELD OF THE DISCLOSURE

This disclosure relates to the therapeutic field of treating intestinal or pulmonary disease, especially in subjects affected with a gut dysbiosis.

### BACKGROUND OF THE DISCLOSURE

The gastrointestinal tract represents the largest compartment of the immune system. It is exposed to food, commensal antigens, and is the portal of entry for many pathogens. One hundred trillion organisms (mainly bacteria, phages but also archae, fungi and parasites) collectively referred to as the gut microbiota colonize the human intestine. The intestinal microbiota comprises microbial populations that colonize the human gastrointestinal tract at increasing densities (from the top to bottom) and has been shown to play a crucial role in human health. Indeed, the gut microbiota exerts a myriad of fundamental functions, such as the degradation of nutrients to provide energy source, the elimination of xenobiotics, the education of the immune system, the growth and terminal differentiation of epithelial cells, the intestinal peristalsis, and the production of antimicrobial peptides to eradicate pathogens and ensure colonization resistance.

Thus, the symbiosis between the intestinal microbiota and the host is a determining parameter for health. Intestinal inflammatory pathologies, metabolic diseases (obesity and type II diabetes), food allergies, are pathologies of the digestive sphere during which a change of the intestinal microbiota is described. The change of the intestinal microbiota is notably characterized by the loss of founding bacteria, a decrease in biodiversity, a lower concentration of bacterial metabolites (such as acetate), a low bacterial gene count and the emergence of opportunistic species like *Clostridium difficile.* A change in the composition / activity of the intestinal microbiota is also described in extra-digestive pathologies, such as respiratory allergies, pulmonary infections or pathologies of the autism spectrum. Maintaining the richness and activity of the intestinal microbiota is therefore at the heart of therapeutic and / or prophylactic intervention strategies in digestive and extra-digestive pathologies.

Perturbations of the symbiosis between the gut microbiome, the intestinal epithelium, and the host immune system are associated with various immuno-pathologies and chronic inflammation. Hence, the composition of the intestinal microbiota has been associated with malnutrition, undernutrition, obesity, and chronic inflammatory disorders such as NASH (Non-Alcoholic SteatoHepatitis) and IBD (Inflammatory bowel disease). It has become evident that the intestinal microbiota not only mediates resistance to colonization by pathogens but also modulates immune function by promoting differentiation of different T-cell phenotypes, which may account for autoimmune or atopic disorders such as rheumatoid arthritis and asthma.

When dysbiosis is associated with the presence of pathogens, an obvious strategy to get rid of health-detrimental microorganisms is the application of antibiotic agents. However, widespread and improper use of broad-spectrum antibiotics over the last decades has dramatically increased antibiotic resistance (Brandl et al., 2008, Nature, Vol. 455:804-7.).

For a therapeutic application, a severely disrupted gut microbiome would benefit more from the introduction of key microbial species, rather than the provision of substrates that benefit health-promoting species that are less abundant or even absent in a diseased individual. A possible solution is the introduction of viable, health-promoting microorganisms, termed probiotics (Iannitti and Palmieri, 2010, Clin. Nutr., Vol. 29:701-25). However, survival of probiotic strains during the harsh conditions of the upper digestive tract is challenging and competition with the vast indigenous microbiome is often negligible. Yet, the concept of introducing new species in a compromised gut ecosystem has gained momentum in recent years through the application of fecal microbial transplants (FMT) (Khoruts et al. 2010, J. Clin. Gastroenterol., Vol. 44:354-60.). FMT seems to efficiently work where single probiotic strains frequently fails. Yet, the badly characterized nature of fecal transplants comes with transmission risks of infectious diseases and currently raises questions over its widespread applicability in less acute and life-threatening pathologies (De Vrieze 2013, Science, Vol. 341:954-7.).

Early 2013, an alternative for fecal microbial transplants entered the field with the publication of a scientific paper (Petrof et al, 2013, Microbiome, Vol. 1:3-10.) and patent application WO2013037068 on the use of a synthetic mixture of microbes, that were isolated from an individual based on their culturability. More recently, it has been suggested in the patent application WO 2017/218680 to make use of compositions comprising various combinations of bacterial strains having bactericidal activity against *C. difficile,* for the purpose of treating or preventing pathogenic infections, especially those caused by *C. Difficile.* The same kind of approach in selecting culturable enteric bacterial strains with the view of treating an infection caused by *C. difficile* is disclosed in the patent application WO 2017/182796. Methods for treating subjects affected with a dysbiosis caused by *C. difficile,* which make use of combinations of bacterial strains originating from the gut microbiota are also disclosed in WO 2017/035188.

There is still a need to design alternative and specific bacterial species or mixtures of bacterial species which can be effectively used to prevent or treat disorders that may be occur in mammals, and especially humans, affected with a gut dysbiosis. There is notably a need for bacterial species or mixtures of bacterial species which can be effectively used for preventing or treating intestinal or pulmonary diseases occurring in subjects affected with a gut dysbiosis.

### SUMMARY OF THE DISCLOSURE

The present disclosure relates to a composition for use in a method for treating and/or preventing intestinal and/or pulmonary disease associated with gut dysbiosis in a subject in need thereof, wherein the composition comprises, in a physiologically acceptable condition, at least one acetate-overproducing bacteria hypersensitive to oxygen (AOHO bacteria).

In some embodiments the said at least one AOHO bacteria is characterized by a 16S rDNA having at least 90% nucleic acid identity with the nucleic acid sequence of SEQ ID NO. 1.

In some embodiments the intestinal and/or pulmonary disease associated with gut dysbiosis is selected in the group comprising : cancer, periodontitis, inflammatory bowel disease, chronic fatigue syndrome, obesity, bacterial vaginosis, colitis, type II diabetes, allergies and frailty syndromes.

In some embodiments the gut dysbiosis is caused by impaired microbiota, antibiotic treatment, medicine, chemotherapies, alcohol, inappropriate diet, undernutrition, mal-nutrition, or virus.
In some embodiments the composition is formulated for oral or rectal administration.
In some embodiments the composition is in the form of a powder or granules, a food product, a beverage, a pharmaceutical, a nutraceutical, a food additive, a food supplement, a dairy product or a capsule.

The present disclosure further relates to a bacterial strain having one or more of the following features :
a. a 16S rDNA having at least 90% nucleic acid identity with the nucleic acid sequence of SEQ ID NO. 1,
b. it is hypersensitive to oxygen, and
c. it is an acetate-overproducing bacteria.
In some embodiments the bacterial strain produces at least 20mmol/L (mM) and at most 100 mM acetate.

### BRIEF DESCRIPTION OF THE FIGURES

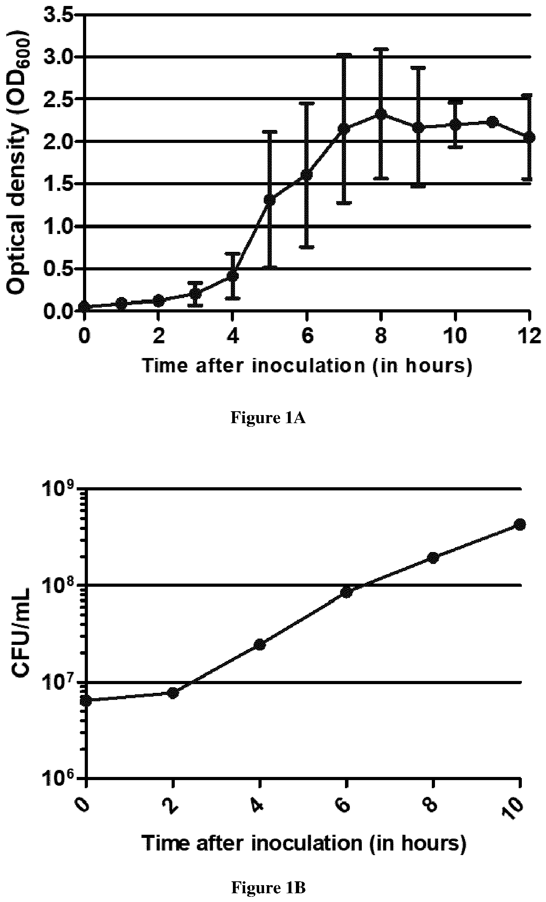

**Figure 1****.** Growth of MB9 strain in BHIS medium. **Figure 1A** : Growth curve of MB9 strain grown in BHIS medium (n=4). Abscissa : Time after inoculation as expressed in hours. Ordinate : optical density as expressed as OD at 600 nanometers (nm). **Figure 1B** : Colony Forming Units (CFUs) count of MB9 strain in BHIS (n=1). Abscissa : Time after inoculation as expressed in hours. Ordinate : CFUs/mL.

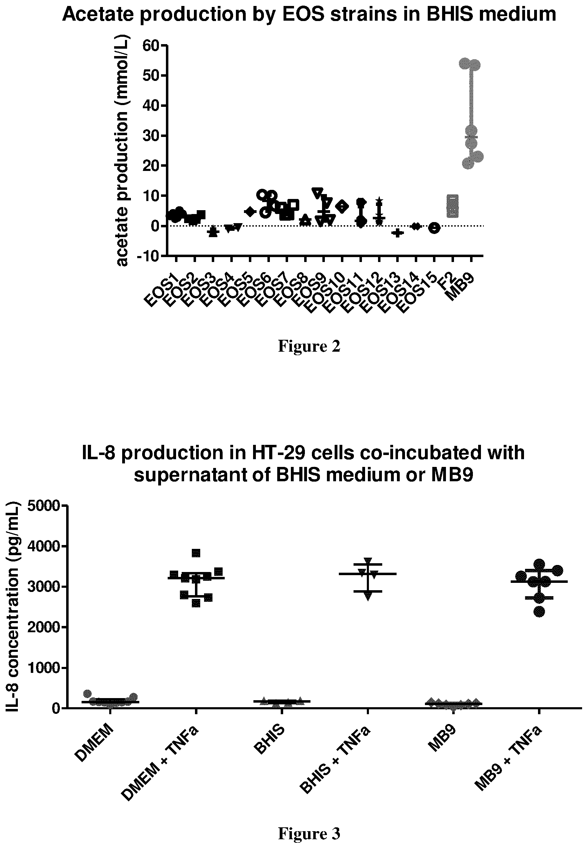

**Figure 2****.** Acetate production by various commensal strains hypersensitive to oxygen after 11 to 17 hours of growth in BHIS. Values were normalised with the basal level of acetate in the BHIS medium. Abscissa : strain names (EOS 1 to 15, F2 and MB9). Ordinate : acetate production as expressed in mmol/L.
**Figure 3****.** IL-8 production in HT-29 cells co-incubated with supernatant of BHIS medium alone or with MB9 strain. Abscissa, from left to right in the figure : (i) DMEM medium alone, (ii) DMEM with TNF alpha, (iii) BHIS medium alone (10% in DMEM), (iv) BHIS medium with TNF alpha (10% in DMEM), (v) MB9 strain in BHIS culture supernatant (10% in DMEM), (vi) MB9 strain in BHIS culture supernatant with TNF alpha (10% in DMEM).

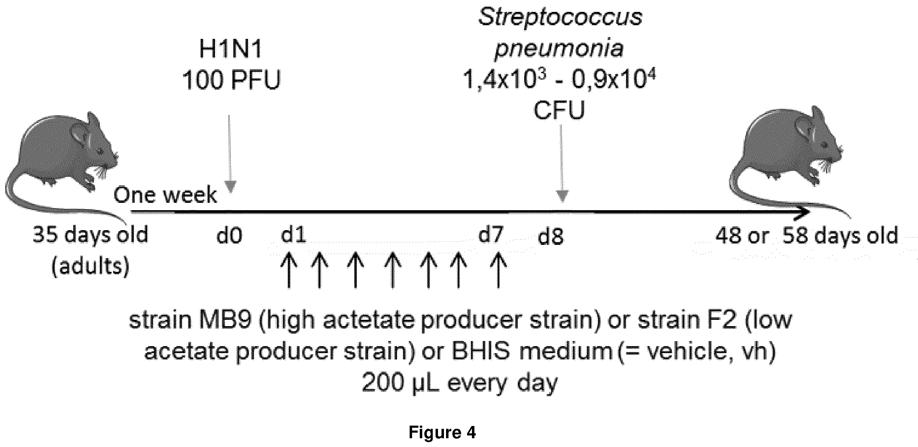

**Figure 4****.** Schedule of the *in vivo* experimental protocol practiced on mice (A.2 *Infections, treatment with MB9 or F2 and assessment of bacterial loads).*

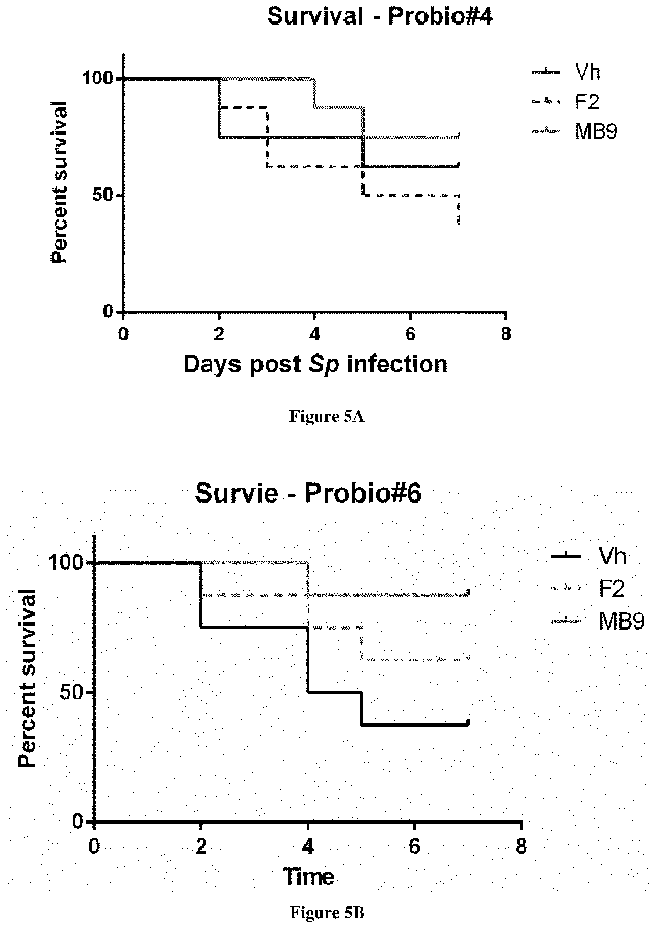

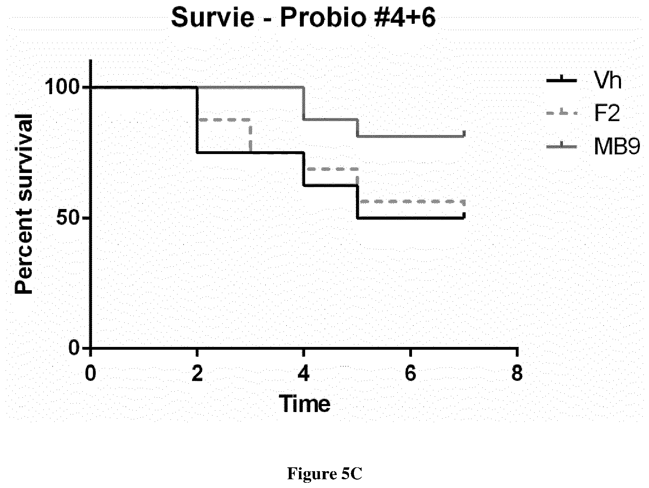

**Figure 5****.** Survival of mice after being super infected with different doses of *Streptococcus pneumoniae.* **Figure 5A** : Results of the Probio#4 experiment; Abscissa : Days post infection with *Streptococcus pneumoniae.* Ordinate : percent survival. **Figure 5B** : Results of the Probio#6 experiment; Abscissa : survival time as expressed in days. Ordinate : percent survival. **Figure 5C** : Average result of the Probio#4+6 experiment; Abscissa : survival time as expressed in days. Ordinate : percent survival.

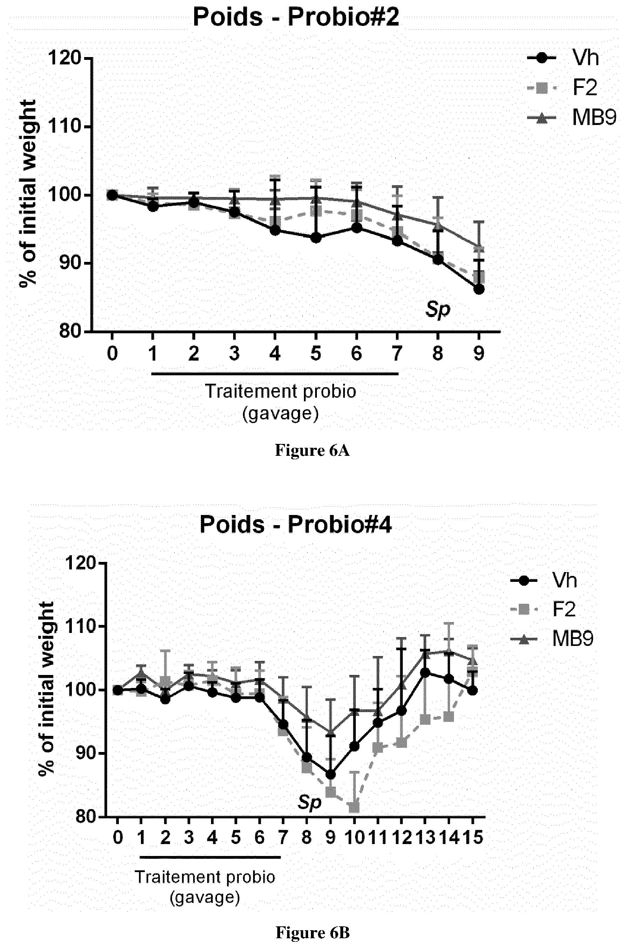

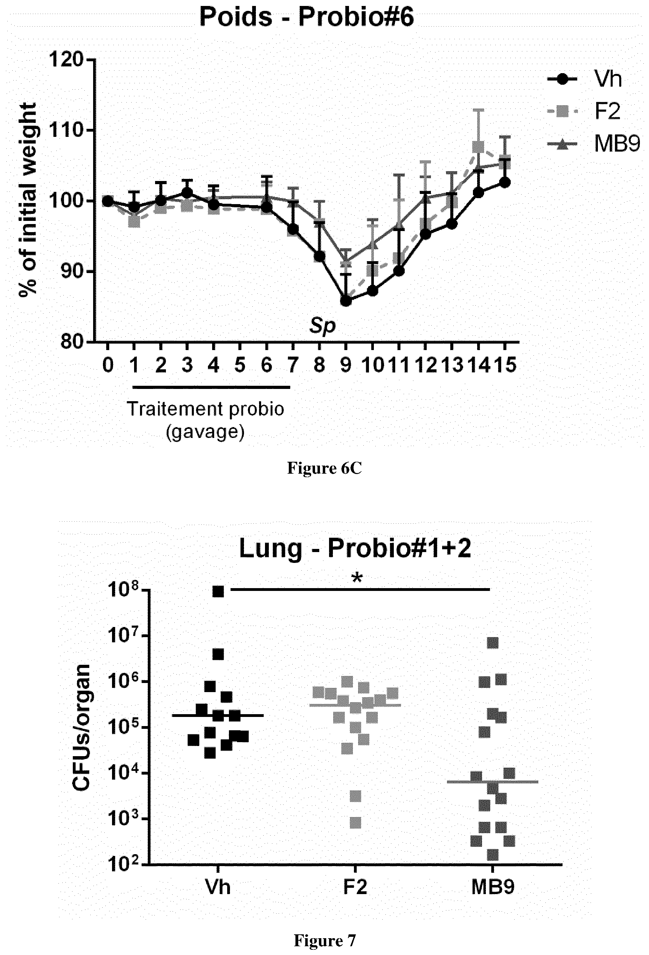

**Figure 6****.** Weight of the mice during and after the gavaging period. **Figure 6A to 6C****.** Abscissa : time period as expressed in days;
   Horizontal line in **Figure 6A** : time period of gavaging (i) "Vh," : with the control BHIS medium at 200 µL daily without any bacterial strain "●", (ii) "F2" : with 4.2x10⁵ CFUs/200µL daily of the F2 strain "■" and (i) "MB9" : with 5.6x10⁶ CFUs/200µL daily of the MB9 strain "▲". Superinfection with 2x10³/30µL (i.n.) *S*. *pneumoniae* per mouse. Ordinate : percent of the initial weight at Day 0.
   Horizontal line in **Figure 6B** **:** time period of gavaging (i) "Vh," : with the control BHIS medium at 200 µL without any bacterial strain "●", (ii) "F2" : with 2x10⁷ CFUs/200µL daily of the F2 strain "■" and (i) "MB9" : with 1.2x10⁵ CFUs/200µL daily of the MB9 strain "▲". Superinfection with 2x10³/30µL (i.n.) *S. pneumoniae* per mouse. Ordinate : percent of the initial weight at Day 0.
   Horizontal line in Figure 6C : time period of gavaging (i) "Vh," : with the control BHIS medium at 200 µL without any bacterial strain "●", (ii) "F2" : with 2.2x10⁷ CFUs/200µL daily of the F2 strain "■" and (i) "MB9" : with 6.8x10⁶ CFUs/200µL daily of the MB9 strain "▲". Superinfection with 0.9x10⁴/30µL (i.n.) *S. pneumoniae* per mouse. Ordinate : percent of the initial weight at Day 0.
**Figure 7****.** Bacterial counts of *Streptococcus pneumoniae* in the lung (in both Probio#1+2) Abscissa : (i) Vh : mice gavaged with control BHIS medium at 200 µL daily without any bacterial strain (ii) mice gavaged daily with 2x10⁷ CFUs/200 µL of the strain F2 (Probio#1) + mice gavaged daily with 4.2x10⁵ CFUs/200 µL of the strain F2 (Probio#2) and, (iii) mice gavaged daily with 1.2x10⁵ CFUs/200 µL of the strain MB9 (Probio#1) + mice gavaged daily with 5.6x10⁶ CFUs/200 µL of the strain MB9 (Probio#2). Ordinate : number of CFUs per lung.

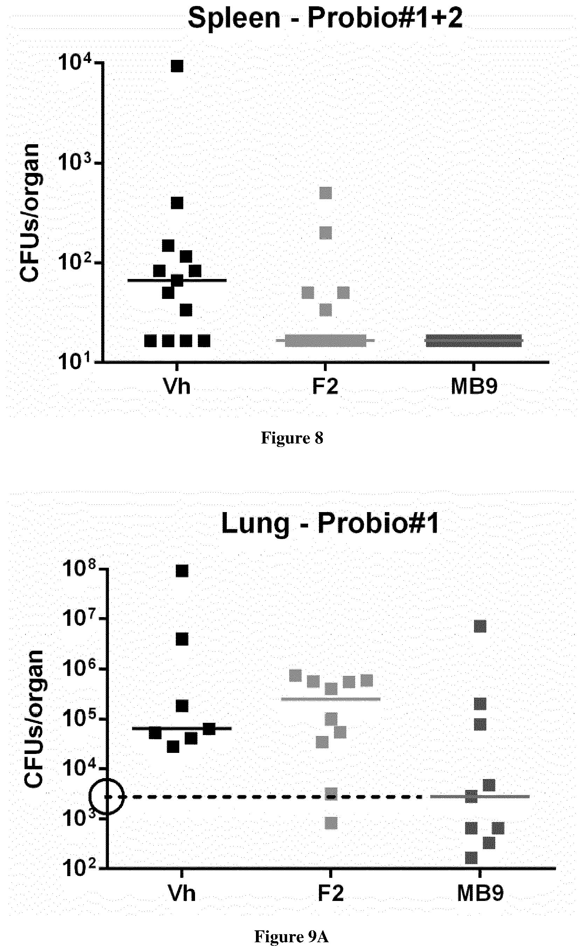

**Figure 8****.** Bacterial counts of *Streptococcus pneumoniae* in the spleen (in both Probio#1+2). Abscissa : (i) Vh : mice gavaged with control BHIS medium at 200 µL daily without any bacterial strain, (ii) F2 : mice gavaged daily with strain F2, (iii) mice gavaged daily with strain MB9. Ordinate : number of CFUs per spleen.

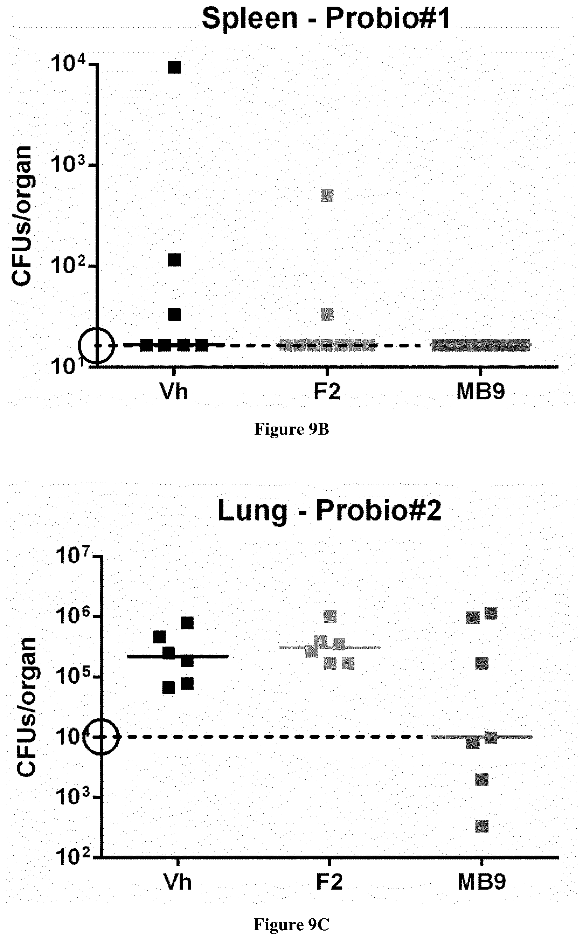

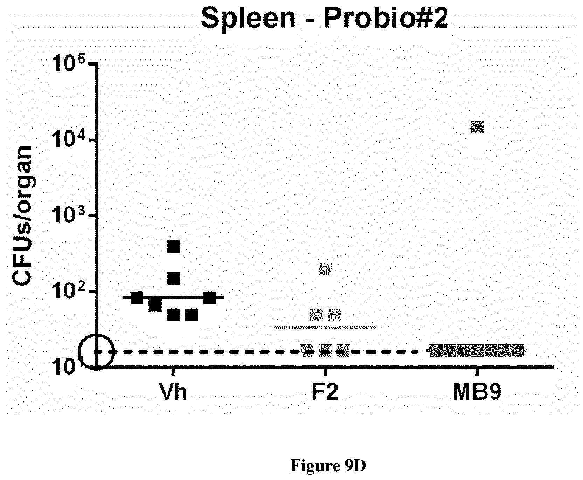

**Figure 9****.** Dose-dependency of the *in vivo* effect of strain MB9.
**Figure 9A** : Bacterial counts of *Streptococcus pneumoniae* in the lung. Vh : mice gavaged with the control BHIS medium at 200µL daily without any bacterial strain, (ii) F2 : mice gavaged with strain F2 at 2x10⁷ CFUs/200 µL daily, (iii) mice gavaged with strain MB9 at 1.2 x 10⁵ CFUs/200 µL daily. Ordinate : number of CFUs per lung.
**Figure 9B** : Bacterial counts of *Streptococcus pneumoniae* in the spleen. Abscissa : (i) Vh : mice gavaged with the control BHIS medium at 200 µL daily without any bacterial strain, (ii) F2 : mice gavaged with strain F2 at 2x10⁷ CFUs/200 µL daily, (iii) mice gavaged with strain MB9 at 1.2 x 10⁵ CFUs/200 µL daily. Ordinate : number of CFUs per spleen.
**Figure 9C** : Bacterial counts of *Streptococcus pneumoniae* in the lung. Vh : mice gavaged with the control BHIS medium at 200 µL daily without any bacterial strain, (ii) F2 : mice gavaged with strain F2 at 4.2x10⁵ CFUs/200 µL daily, (iii) mice gavaged with strain MB9 at 5.6x10⁶ CFUs/200 µL daily. Ordinate : number of CFUs per lung.
**Figure 9D** : Bacterial counts of *Streptococcus pneumoniae* in the spleen. Abscissa : (i) Vh : mice gavaged with the control BHIS medium at 200 µL daily without any bacterial strain, (ii) F2 : mice gavaged with strain F2 at 4.2x10⁵ CFUs/200 µL daily, (iii) mice gavaged with strain MB9 at 5.6x10⁶ CFUs/200 µL daily. Ordinate : number of CFUs per spleen.

### DETAILED DESCRIPTION OF THE DISCLOSURE

The present inventors have selected bacteria originating from the human fecal sample representing gut microbiome that are able of overcoming the negative effects caused by the occurrence of a gut dysbiosis in a subject.

Importantly, the inventors have shown that these selected bacteria stimulate lung immunity, as it is illustrated in subjects infected with an *influenza* virus, wherein the said selected bacteria enhance pulmonary immune defenses against a secondary infection, especially against a secondary pulmonary infection, such as with *S. pneumoniae.* The said selected bacteria also generally enhance systemic immunity, as it is illustrated by a reduced presence of pathogenic bacteria in spleen in virus-infected subjects exposed to a superinfection with such pathogenic bacteria, like *S. pneumoniae.*

As it is shown herein, administration of these selected bacteria enhances the survival rate of double-infected subjects, preferably a mammal, more preferably a human.

It has also been shown that these selected bacteria protect a subject against some negative physiological consequences caused by an infection, especially by a viral infection such as by an *influenza* virus. As shown in the examples herein, administration of these selected bacteria to a virus-infected subject, such as a subject infected with an *influenza* virus, protects the said subject against the weight loss caused by the said viral infection.

Accordingly, it is disclosed herein the use of the bacteria selected by the inventors for the purpose of treating or preventing intestinal and/or pulmonary diseases in a subject in need thereof, and especially in a subject affected with a gut dysbiosis.

### Definitions

As used herein, the term gut or intestinal "dysbiosis" refers to an imbalance of a subject's gut microbiome. The term gut "dysbiosis" as used herein refers to a state of the microbiota of the gut in a subject, in which the normal diversity and/or function of the microbial populations is disrupted. This unhealthy state can be due to a decrease in diversity, the overgrowth of one or more pathogens or pathobionts, symbiotic organisms able to cause disease only when certain genetic and/or environmental conditions are present in a subject, or the shift to an ecological microbial network that no longer provides an essential function to the host subject, and therefore no longer promotes health. According to non- limitative examples, essential functions may include enhancement of the gut mucosal barrier, direct or indirect reduction and elimination of invading pathogens, enhancement of the absorption of specific substances, maturation of immune system and suppression of gastrointestinal inflammation. Any disruption from the preferred (*e.g.,* ideal) state of the microbiota can be considered a dysbiosis, even if such dysbiosis does not result in a detectable decrease in health. This state of dysbiosis may be unhealthy, it may be unhealthy under only certain conditions, or it may prevent a subject from becoming healthier. Dysbiosis may be due to a decrease in diversity, a decrease in gene count, a decrease in Short Chain Fatty Acids (and more generally a loss of metabolic functions) the overgrowth of one or more pathogens or pathobionts, symbiotic organisms able to cause disease only when certain genetic and/or environmental conditions are present in a patient, or the shift to an ecological network that no longer provides a beneficial function to the host and therefore no longer promotes health.

As used herein, the term "Short Chain Fatty Acids" or "SCFAs" is commonly understood in the art and refers to fatty acids with fewer than six carbon atoms. Particularly SCFAs encompass formic acid, acetic acid, propionic acid, butyric acid, isobutyric acid, valeric acid, isovaleric acid or its salts such as formate, acetate, propionate, butyrate, isobutyrate, valerate and isovalerate.

In the human fecal samples, the proportion of Extremely Oxygen Sensitive (EOS) strains is positively correlated to the amount of fecal acetate (Martin et al., Front Microbiol. 2017 Jun 30;8:1226. doi: 10.3389/fmicb.2017.01226. eCollection 2017.PMID:28713353) .

As used herein, the term "microbiome" refers to all the bacteria, fungi, protozoa and virus in a community and specific environment. As a non-limiting example, the human microbiome includes all the bacteria, fungi, protozoa and virus that live on and inside the subject body, *i.e.* human body. The term "gut microbiome" as used herein refers to all the bacteria, fungi, protozoa and virus that live in the subject's gut, preferably the human's gut. These microorganisms, mainly comprising bacteria, are involved in functions critical to the health and wellbeing of the subject, *i.e.* human. These bacteria live in the digestive system of the subject.

As used herein, the term "subject" refers to a mammal and preferably to a human. "Subject" encompasses subjects older than 1 year old and less than 14 years old (*i.e.* children); adults, subjects between the ages of 50 and 65, and adults who are older than 65 years of age.

Within the scope of the present disclosure, the "percentage identity" between two sequences of nucleic acids means the percentage of identical nucleotides residues between the two sequences to be compared, obtained after optimal alignment, this percentage being purely statistical and the differences between the two sequences being distributed randomly along their length. The comparison of two nucleic acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970), by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr. Madison, WI, or by the comparison software BLAST NR or BLAST P).

The percentage identity between two nucleic acid sequences is determined by comparing the two optimally-aligned sequences in which the nucleic acid sequence to compare can have additions or deletions compared to the reference sequence for optimal alignment between the two sequences. Percentage identity is calculated by determining the number of positions at which the nucleotide residue is identical between the two sequences, preferably between the two complete sequences, dividing the number of identical positions by the total number of positions in the alignment window and multiplying the result by 100 to obtain the percentage identity between the two sequences.

As intended herein, nucleotide sequences having at least 90% nucleotide identity with a reference sequence encompass those having at least 91%, 92%, 93%, 94%, 95%, 96%, 97%, 98% and 99% nucleotide identity with the said reference sequence.

As used herein, bacteria "hypersensitive to oxygen" consist of bacteria having a maximum survival time, when cultured in aerobic conditions, of 10 minutes. The bacterial survival time in aerobic conditions is assessed according to the method according to "Oxygen sensitivity determination" in the material and methods of the present disclosure.

As used herein, bacteria consist of "acetate-overproducing bacteria" when the said bacteria produce 20 mmol/L after 11-17 hours of growth in BHIS or more acetate when cultured in the following conditions : 10⁸ to 10⁹ CFUs/mL of bacteria after 11-17 hours of growth. The amount of acetate in the culture supernatant is measured according to the method discloses in "Short-chain-fatty-acid (SCFA) production and consumption of the EOS strains, grown in BHIS" in the material and methods of the present disclosure.

As used herein, a "16S rDNA" consists of a DNA nucleic acid, the nucleic acid sequence of which is fully complementary to the naturally occurring 16S rRNA. A 16S rDNA may be sequenced by using the primers fD1 (SEQ ID NO. 2 herein) and rp2 (SEQ ID NO. 3 herein) (Weisburg *et al.* 1991 Weisburg, W. G., S. M. Barns, D. A. Pelletier, and D. J. Lane.1991. 16S ribosomal DNA amplification for phylogenetic study. J. Bacteriol.173:697-703) and Dreamtaq polymerase (Thermofisher) for amplification of RNA from a pure colony.

As used herein, "16S rRNA" is well known in the art and consists of the component of the 30S small subunit of a prokaryotic ribosome that is known for binding the Shine-Dalgarno sequence.

As used herein, "frailty syndromes" is a geriatric syndrome that reflects the state of decreased physiologic reserves and vulnerability to stressors related to accelerate aging. The pathophysiology of frailty is rooted in dysregulation of multiple inter-related systems, particularly the immune, hormonal, and endocrine systems.

As used herein, the "inflammatory bowel disease" encompasses ulcerative colitis and/or Crohn's disease.

As used herein, the term "allergies" or "allergic diseases" refers to a number of diseases conditions caused by hypersensitivity of the subject immune system to typically harmless substances in the environment. Allergies include pollen allergy (hay fever), food allergies, atopic dermatitis, allergic asthma, and anaphylaxis. Symptoms may include red eyes, an itchy rash, sneezing, a runny nose, shortness of breath or swelling.

### Selected bacteria according to the present disclosure

The inventors have selected specific bacteria initially originating from the human gut microbiome for their properties of enhancing the defenses of a subject against bacterial infections, including against bacterial superinfections in subjects already infected with a virus such as an *influenza* virus.

According to some embodiments, the said selected bacteria are hypersensitive to oxygen.

According to some embodiments, the said selected bacteria are high producer of acetate.

The selected bacteria according to the present disclosure may also be termed "AOHO" bacteria (for "Acetate-Overproducing bacteria Hypersensitive to Oxygen").

The selected AOHO bacteria according to the present disclosure may be characterized in that their 16S rDNA has 90% nucleic acid identity or more with the nucleic acid of SEQ ID NO. 1 disclosed herein. Thus, the selected bacteria encompass those for which the 16S rDNA has at least 91%, 92%, 93%, 94%, 95%, 96%, 97%, 98% and 99% nucleotide identity with the nucleic acid sequence of SEQ ID NO. 1.

In some embodiments, the selected AOHO bacteria according to the present disclosure encompasses bacteria having a 16S rDNA with 100% acid nucleic identity with the nucleic acid of SEQ ID NO. 1 disclosed herein.

Without wishing to be bound by any particular theory, it is believed that the inventors' findings have allowed defining a family of closely related bacteria having closely related genetic features, especially having a close 16S rDNA sequence, which closely related genetic features determine the same functional features, such as determining the common ability to enhance the systemic and/or the pulmonary immune responses in subjects affected with a bacterial infection, which encompasses subjects affected with a bacterial superinfection following a viral infection such as an infection with an *influenza* virus.

The selected AOHO bacteria may be grown by culture in the BHIS medium (Brain Heart Infusion medium Supplemented with yeast extract 0.5% broth) in the culture conditions disclose in "Bacterial culture of MB9" in the material and methods of the present disclosure.

In some embodiments, the selected AOHO bacteria consist of the MB9 strain that has been deposited in the name of Institut National de la Recherche Agronomique (INRA) according to the Budapest Treaty at the Collection DSMZ-German Collection of Microorganisms and Cell Cultures GmbH on December 19th, 2019 under the reference number DSM 33383.

As used herein, the term "treatment" or "treat" refers to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment.

The compositions and methods of the present disclosure, which make use of the AOHO bacteria disclosed herein, are particularly suitable for subjects who are identified as at high risk for developing a bacterial superinfection post-influenza, including subjects who are at least 50 years old, subjects who reside in chronic care facilities, subjects who have chronic disorders of the pulmonary or cardiovascular system, subjects who are under or malnourished (suffering from malnutrition), subjects with long-term treatments like antibiotics and chemotherapies, frailty subjects, subjects who required regular medical follow-up or hospitalization during the preceding year because of chronic metabolic diseases (including diabetes mellitus), renal dysfunction, hemoglobinopathies, or immunosuppression (including immunosuppression caused by medications or by human immunodeficiency [HIV] virus); children less than 14 years of age, patients between 6 months and 18 years of age who are receiving long-term aspirin therapy, and women who will be in the second or third trimester of pregnancy during the influenza season. More specifically, it is contemplated that the method of the disclosure is suitable for the treatment of bacterial superinfection post-influenza in subjects older than 1 year old and less than 14 years old (*i.e.,* children); subjects between the ages of 50 and 65, and adults who are older than 65 years of age.

### Uses of the selected bacteria

As it is illustrated in the examples herein, the selected AOHO bacteria that have been shown to enhance both systemic and pulmonary immune responses against a bacterial infection, including a bacterial superinfection, may be effectively used for therapeutic purposes since these selected AOHO bacteria, when administered to a subject in need thereof, will not simultaneously induce pro-inflammatory responses. Notably, the inventors have shown that the selected bacteria did not inhibit IL-8 production in human HT-29 cells incubated in the presence of TNF-alpha (figure 3).

Without wishing to be bound by any particular theory, the inventors believe that the property of the selected AOHO bacteria to produce high amount of acetate may contribute to some extent to their ability of enhancing the subject's immune responses against a bacterial infection, which encompasses enhancing the subject's immune responses against a bacterial superinfection in subjects already infected with a virus such as infected with an *influenza* virus.

Without wishing to be bound by any particular theory, the inventors believe that the property of the selected AOHO bacteria to be hypertensive to oxygen and thus to be adapted to grow in anaerobic or substantially anaerobic conditions is particularly advantageous, since this specific property shall ensure their survival and favorize their colonization of the gut, which consists of an anaerobic environment. Further, this bacteria should have a privileged tropism for intestinal tract, since it has been isolated from fecal microbiota.

The present disclosure relates to at least one acetate-overproducing bacteria hypersensitive to oxygen (AOHO bacteria) for use in a method for treating and/or preventing intestinal and/or pulmonary disease in a subject in need thereof.

In some preferred embodiments, the at least one acetate-overproducing bacteria hypersensitive to oxygen (AOHO) is an intestinal commensal bacteria.

As used herein, the term "intestinal commensal" bacteria refers to bacteria that are found in the subject gut microbiome.

The present disclosure also relates to a composition for use in a method for treating and/or preventing intestinal and/or pulmonary disease in a subject in need thereof, wherein the composition comprises, in a physiologically acceptable condition, at least one acetate-high producer intestinal bacteria hypersensitive to oxygen (AOHO bacteria).

The present disclosure further relates to a composition for use in a method for treating and/or preventing intestinal and/or pulmonary disease in a subject in need thereof, wherein the composition comprises, in a physiologically acceptable condition, an effective amount of at least one AOHO bacteria.

In preferred embodiments, the intestinal and/or pulmonary disease is associated with a gut dysbiosis.

Thus, in some preferred embodiments, the present disclosure relates to a composition for use in a method for treating and/or preventing intestinal and/or pulmonary disease associated with a gut dysbiosis in a subject in need thereof, wherein the composition comprises, in a physiologically acceptable condition, an effective amount of at least one AOHO bacteria.

This disclosure further relates to the use of at least one AOHO bacteria for preparing a medicament for treating and/or preventing intestinal and/or pulmonary disease in a subject in need thereof.

This disclosure relates to the use of at least one AOHO bacteria for preparing a medicament for treating and/or preventing intestinal and/or pulmonary disease associated with a gut dysbiosis in a subject in need thereof.

This disclosure pertains to a method for treating and/or preventing intestinal and/or pulmonary disease in a subject in need thereof, comprising a step of administering to the said subject at least one AOHO bacteria.

This disclosure further pertains to a method for treating and/or preventing intestinal and/or pulmonary disease associated with a gut dysbiosis in a subject in need thereof, comprising a step of administering to the said subject at least one AOHO bacteria.

This disclosure also concerns a method for treating and/or preventing intestinal and/or pulmonary disease in a subject in need thereof, comprising a step of administering to the said subject a composition comprising at least one AOHO bacteria.

This disclosure further concerns a method for treating and/or preventing intestinal and/or pulmonary disease associated with a gut dysbiosis in a subject in need thereof, comprising a step of administering to the said subject a composition comprising at least one AOHO bacteria.

In most preferred embodiments, the selected AOHO bacteria is characterized by a 16S rDNA having at least 90% nucleic acid identity with the nucleic acid sequence of SEQ ID NO. 1.

In some embodiments of the said composition, the AOHO bacteria in the composition are present in a range of 10³ to 10⁹ CFUs per dose unit.

In some embodiments, the intestinal and/or pulmonary disease associated with gut dysbiosis is selected in a group comprising : cancer, periodontitis, inflammatory bowel disease, chronic fatigue syndrome, obesity, bacterial vaginosis, colitis, type II diabetes, allergies and frailty syndromes.

In some embodiments, the gut dysbiosis is caused by impaired microbiota, antibiotic treatment, medicine, chemotherapies, alcohol, inappropriate diet, undernutrition, mal-nutrition, or virus.

In some embodiments, the composition allows achieving weight management in a subject, especially in a subject infected by a virus, such as a subject infected with an *influenza* virus. More precisely, the composition allows reducing the weight loss in subjects infected with a virus such as an *influenza* virus, which encompasses virus-infected subjects, which includes subjects infected with an *influenza* virus, undergoing a bacterial superinfection such as a superinfection by *S. pneumoniae.*

Thus, according to some embodiments, the subject suffers or has suffered from an *influenza* infection.

As used herein, the term *"influenza* infection" has its general meaning in the art and refers to the disease caused by an infection by an *influenza* virus. In some embodiments, *influenza* infection is associated with *Influenza* virus A or B. In some embodiments, *influenza* infection is associated with *Influenza* virus A. In some embodiments, *influenza* infection is caused by *influenza* virus A that is H1N1, H2N2, H3N2 or H5N1.

As used herein, the expression "bacterial superinfection *post-influenza"* has its general meaning in the art and refers to a bacterial infection (e.g. bacterial pneumonia) which occurs in a subject who suffers or has suffered from an *influenza* infection. Typically, the bacterial superinfection occurs within 4, 5, 6, 7, 8, 9, 10, 11, 12, 13, 14, 15, 16, 17, 18, 19, 20, 21, 22, 23, 24, or 25 days after *influenza* infection. According to the present disclosure, a selected AOHO bacteria that is disclosed herein is particularly suitable for the treatment of a bacterial superinfection *post-influenza* such as, but not limited to, infections of the lower respiratory tract (*e.g*., pneumonia), middle ear infections (*e.g.,* otitis media) and bacterial sinusitis. The bacterial superinfection may be caused by numerous bacterial pathogens. For example, they may be mediated by at least one organism selected from the group consisting of: *Streptococcus pneumoniae, Staphylococcus aureus, Haemophilus influenza, Myoplasma* species, *Moraxella* species, *Pseudomonas* species and emergent pathogens.

In preferred embodiments, the AOHO bacteria disclosed herein, or a composition comprising at least one AOHO bacteria, is formulated for oral or rectal administration.

In some embodiments, the composition is in the form of a powder or granules, a food product, a beverage, a pharmaceutical, a nutraceutical, a food additive, a food supplement, a dairy product or a capsule.

The present disclosure also concerns the use of at least one AOHO bacteria for treating and/or preventing a bacterial superinfection, preferably a bacterial pulmonary superinfection, in a subject, such as a subject already infected with a virus, such as an *influenza* virus.

The present disclosure further concerns a composition for use in a method for treating and/or preventing a bacterial superinfection, preferably a bacterial pulmonary superinfection, in a subject in need thereof, wherein the composition comprises, in a physiologically acceptable condition, an effective amount of at least one acetate-overproducing bacteria hypersensitive to oxygen (AOHO bacteria).

The present disclosure also concerns the use of at least one AOHO bacteria for preparing a medicament for treating and/or preventing a bacterial superinfection, preferably a bacterial pulmonary superinfection, in a subject, such as a subject already infected with a virus, such as an *influenza* virus.

The present disclosure further concerns a composition for use in a method for treating and/or preventing a bacterial superinfection associated with gut dysbiosis, preferably a bacterial pulmonary superinfection, in a subject in need thereof, wherein the composition comprises, in a physiologically acceptable condition, an effective amount of at least one AOHO bacteria.

The present disclosure also relates to a method for treating and/or preventing a bacterial superinfection associated with gut dysbiosis, preferably a bacterial pulmonary superinfection, in a subject in need thereof, comprising administering to a subject in need thereof a composition comprising in a physiologically acceptable condition, an effective amount of at least one AOHO bacteria.

The present disclosure further relates to a bacterial strain having one or more of the following features :
- it has a 16S rDNA having at least 90% nucleic acid identity with the nucleic acid sequence of SEQ ID NO. 1,
- it is hypersensitive to oxygen,
- it is an acetate-overproducing bacteria, preferably it is an overproduces acetate, *i.e.* at an amount >20 mmol/L, and/or
- it is an intestinal commensal bacteria.

The present disclosure also relates to a bacterial strain having one or more of the following features :
- it has a 16S rDNA having at least 90% nucleic acid identity with the nucleic acid sequence of SEQ ID NO. 1,
- it is hypersensitive to oxygen,
- it is an acetate-overproducing bacteria, preferably it is an overproduces acetate, *i.e.* at an amount >20 mmol/L, and/or
- it is an intestinal commensal bacteria,
for use in a method for treating and/or preventing intestinal and/or pulmonary disease in a subject in need thereof.

The present disclosure further relates to a bacterial strain having one or more of the following features :
- it has a 16S rDNA having at least 90% nucleic acid identity with the nucleic acid sequence of SEQ ID NO. 1,
- it is hypersensitive to oxygen,
- it is an acetate-overproducing bacteria, preferably it is an overproduces acetate, *i.e.* at an amount >20 mmol/L, and/or
- it is an intestinal commensal bacteria,
for use in a method for treating and/or preventing a bacterial superinfection associated with gut dysbiosis, preferably a bacterial pulmonary superinfection, in a subject in need thereof.

The present disclosure further pertains to the AOHO bacterial strain which is termed "MB9" herein, that has been deposited in the name of Institut National de la Recherche Agronomique (INRA) according to the Budapest Treaty at the Collection DSMZ-German Collection of Microorganisms and Cell Cultures GmbH on December 19th, 2019 under the reference number DSM 33383.

### Compositions useful according to the present disclosure

Generally, the selected AOHO bacteria according to the present disclosure may be obtained by using fermentation methods that are well known in the art, and especially by using anaerobic fermenters, which can support the rapid growth of anaerobic bacterial species. The anaerobic fermenters may be stirred tank reactors or disposable wave reactors. After being grown in culture, the resulting selected AOHO bacteria are preferably purified a concentrated from the fermentation broth by traditional methods, such as centrifugation and filtration, and may then be dried, cryopreserved or lyophilized by well-known methods. The selected AOHO bacteria may be cryopreserved or lyophilized according to the methods disclosed by Bircher et al. (2018, Microbial Biotechnology, Vol. 11 (n°4) : 721-733) or according to the method discloses in FR 3045384.

In a composition according to the present disclosure, which includes a food composition or a pharmaceutical composition, the selected AOHO bacteria may be in any form, such as in an aqueous form like a solution or a suspension, embedded in a semi-solid form, in a powdered form or also in a freeze-dried (*i.e.* lyophilized) form. However, the said selected AOHO bacteria are most preferably in a lyophilized form, which form being the most susceptible to maintain viability of these selected bacteria during storage.

In some embodiments, the selected AOHO bacteria may be lyophilized and then included as an ingredient when manufacturing a composition according to the present disclosure.

In some other embodiments, the selected AOHO bacteria may be lyophilized separately and then combined with a composition comprising the other ingredients a short period of time prior to its administration to the subject, *e.g.* two hours or less, or even one hour or less, prior to its administration to the subject.

In most preferred embodiments, irrespective of whether the composition according to the present disclosure consists of a food composition or a pharmaceutical composition, the said composition comprises an amount of AOHO bacteria ranging from 10³ to 10⁹ CFUs per dosage unit.

As used herein, the term "dosage unit" may be used irrespective of whether the composition comprising at least one AOHO bacteria is in the form of a food composition or in the form of a pharmaceutical composition. In embodiments wherein the composition is in the form of a food composition, the "dosage unit" consists of the amount or portion of a food composition with which the subject in need thereof is fed. In embodiments wherein the composition is in the form of a pharmaceutical composition, the term "dosage unit" is used according to its conventional meaning (*e.g.* a pill, a tablet, a capsule, an ampoule, etc.)

In some embodiments, a composition comprising at least one selected acetate-overproducing bacteria hypersensitive to oxygen (AOHO bacteria) consists of a food composition.

In some embodiments, the food composition is selected from complete food compositions, food supplements, nutraceutical compositions, and the like. The composition of the present disclosure may be used as a food ingredient and/or feed ingredient. The food ingredient may be in the form of a solution or as a solid-depending on the use and/or the mode of application and/or the mode of administration. As used herein, the term "food" refers to liquid (*i.e.* drink), solid or semi-solid dietetic compositions, especially total food compositions (food-replacement), which do not require additional nutrient intake or food supplement compositions. Food supplement compositions do not completely replace nutrient intake by other means. As used herein the term "food ingredient" or "feed ingredient" includes a formulation which is or can be added to functional foods or foodstuffs as a nutritional supplement. By "nutritional food" or "nutraceutical" or "functional" food, is meant a foodstuff which contains ingredients having beneficial effects for health or capable of improving physiological functions. By "food supplement", is meant a foodstuff having the purpose of completing normal food diet. A food supplement is a concentrated source of nutrients or other substances having a nutritional or physiological effect, when they are taken alone or as a combination in small amounts. According to the present disclosure, "functional food" summarizes foodstuff and corresponding products lately developed to which importance is attributed not only due to them being valuable as to nutrition and taste but due to particular ingredient substances. In some embodiments, a composition according to the present disclosure typically comprises carriers or vehicles. "Carriers" or "vehicles" mean materials suitable for administration and include any such material known in the art such as, for example, any liquid, gel, solvent, liquid diluent, solubilizer, or the like, which is non-toxic and which does not interact with any components of the composition in a deleterious manner. Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

In some embodiments, the composition comprises any other ingredients or excipients known to be employed in the type of composition in question. Non limiting examples of such ingredients include : proteins, amino acids, carbohydrates, oligosaccharides, lipids, prebiotics or probiotics, nucleotides, nucleosides, other vitamins, minerals and other micronutrients.

In some embodiments, the composition contains a carbohydrate source, preferably as prebiotics, or in addition to prebiotics. Any carbohydrate source conventionally found in infant formulae such as lactose, saccharose, maltodextrin, starch and mixtures thereof may be used although the preferred source of carbohydrates is lactose.

In some embodiments, the composition comprises a probiotic. As used herein the term "probiotic" is meant to designate live microorganisms which, they are integrated in a sufficient amount, exert a positive effect on health, comfort and wellness beyond traditional nutritional effects. Probiotic microorganisms have been defined as "Live microorganisms which when administered in adequate amounts confer a health benefit on the host" (FAO/WHO 2001). Non limiting examples of probiotics include: *Bifidobacterium, Lactobacillus, Lactococcus, Enterococcus, Streptococcus, Kluyveromyces, Saccharoymces, Candida,* in particular selected from the group consisting of *Bifidobacterium longum, Bifidobacterium lactis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus* salivarius, *Lactococcus lactis, Enterococcus faecium, Enterococcus faecalis, Saccharomyces cerevisiae, Saccharomyces boulardii* or mixtures thereof, preferably selected from the group consisting of *Bifidobacterium longum* NCC3001 (ATCC BAA-999), *Bifidobacterium longum* NCC2705 (CNCM 1-2618), *Bifidobacterium longum* NCC490 (CNCM 1-2170), *Bifidobacterium lactis* NCC2818 (CNCM I- 3446), *Bifidobacterium breve* strain A, *Lactobacillus paracasei* NCC2461 (CNCM 1-2116), Lactobacillus *jolmsonii* NCC533 (CNCM 1-1225), *Lactobacillus rhamnosus* GG (ATCC53103), *Lactobacillus rhamnosus* NCC4007 (CGMCC 1.3724), *Enterococcus faecium* SF 68 (NCC2768; NCIMB 10415), and combinations thereof. In an embodiment of the disclosure, the infant formula further includes a probiotic strain such as a probiotic bacterial strain in an amount of from 10⁶ to 10¹¹ CFUs/g of composition (dry weight).

In some embodiments, the composition comprises one or more prebiotic. Non-limiting examples of prebiotics include: oligosaccharides optionally containing fructose, galactose, mannose; dietary fibers, in particular soluble fibers, soy fibers; inulin; and combinations thereof. Preferred prebiotics are fructo-oligosaccharides (FOS), galacto-oligosaccharides (GOS), isomalto-oligosaccharides (IMO), xylo-oligosaccharides (XOS), arabino-xylo oligosaccharides (AXOS), mannan-oligosaccharides (MOS), oligosaccharides of soy, glycosylsucrose (GS), lactosucrose (LS), lactulose (LA), palatinose-oligosaccharides (PAO), malto-oligosaccharides, gums and/or hydrolysates thereof, pectins and/or hydrolysates thereof, and combinations of the foregoing.

In one embodiment, the composition comprises one or more vitamins. Vitamins may be folic acid, vitamin B 12 and vitamin B6, in particular folic acid and vitamin B12, in particular folic acid. In some embodiments, the composition comprises one or more vitamin which is lipid-soluble, for example one or more of vitamin A, vitamin D, vitamin E and vitamin K.

In some embodiments, the composition further comprises one or more mineral. Examples of minerals are sodium, potassium, chloride, calcium, phosphate, magnesium, iron, zinc, copper, selenium, manganese, fluoride, iodine, chromium, or molybdenum. The minerals are usually added in salt form. The minerals may be added alone or in combination.

In some embodiments, the composition is a fermented dairy product or dairy-based product, which is preferably administered or ingested orally one or more times daily. Fermented dairy products include milk-based products, such as (but not limited to) deserts, yoghurt, yoghurt drinks, quark, kefir, fermented milk-based drinks, buttermilk, cheeses, dressings, low fat spreads, fresh cheese, soy-based drinks, ice cream, etc. Alternatively, in some embodiments, food and/or food supplement compositions may be non-dairy or dairy non fermented products (e.g. strains or cell-free medium in non-fermented milk or in another food medium). Non- fermented dairy products may include ice cream, nutritional bars and dressings, and the like. Non-dairy products may include powdered beverages and nutritional bars, and the like. The products may be made using known methods, such as adding an effective amount of an AOHO bacteria according to the present disclosure to a food base, such as skimmed milk or milk or a milk-based composition and fermentation as known. In some embodiments, the composition is a drink that can be a functional drink or a therapeutic drink, a thirst-quencher or an ordinary drink. By way of example, the composition of the present disclosure can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.

In some other embodiments, the at least one acetate-overproducing bacteria hypersensitive to oxygen (AOHO bacteria) according to the present disclosure is administered to the subject in a form of a pharmaceutical composition. For instance, the at least one AOHO bacteria according to the present disclosure may be combined with pharmaceutically acceptable excipients, and optionally sustained-release matrices, such as biodegradable polymers, to form therapeutic compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. In the pharmaceutical compositions of the present disclosure for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists.

The pharmaceutical composition must be stable under the conditions of manufacture and storage and must be preserved against the contaminating action of microorganisms, such as bacteria and fungi. Solutions comprising compounds of the disclosure as free base or pharmacologically acceptable salts can be prepared in water suitably mixed with a surfactant, such as hydroxypropyl cellulose. Dispersions can also be prepared in glycerol, liquid polyethylene glycols, and mixtures thereof and in oils. Under ordinary conditions of storage and use, these preparations contain a preservative to prevent the growth of microorganisms. The at least one AOHO bacteria according to the present disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

As used herein, the term "therapeutically effective amount" is an equivalent phrase refers to the amount of a therapy (*e.g.,* a prophylactic or therapeutic agent), which is sufficient to reduce the severity and/or duration of a disease, ameliorate one or more symptoms thereof, prevent the advancement of a disease or cause regression of a disease, or which is sufficient to result in the prevention of the development, recurrence, onset, or progression of a disease or one or more symptoms thereof, or enhance or improve the prophylactic and/or therapeutic effect(s) of another therapy (e.g., another therapeutic agent) useful for treating a disease.

As it is already disclosed previously herein, a typical effective amount of at least one AOHO bacteria per dosage unit of a composition according to the present disclosure ranges from 10³ to 10⁹ CFUs per dosage unit.

The present disclosure is further illustrated, without in any way being limited to, by the examples hereafter.

### EXAMPLES

### Example 1 : Specific structural and functional features of an acetate-overproducing commensal bacterial strain : MB9

### A. Materials and Methods

### A.1 Bacterial isolation

The inventors have isolated 17 fecal commensal extremely oxygen sensitive bacteria from healthy humans. Bacteria were isolated according to the method disclosed in the article Martin *et al.,* 2017 (Martin et al., Front Microbiol. 2017 Jun 30;8:1226. doi: 10.3389/fmicb.2017.01226. eCollection 2017.PMID:28713353). All isolated bacteria are named EOS1 to EOS 1, EOS 2, EOS 3, EOS 4, EOS 5, EOS 6, EOS 7, EOS 8, EOS 9, EOS 10, EOS 11, EOS 12, EOS 13, EOS 14, EOS 15, F2 and MB9. MB9 has a surprisingly high acetate overproduction activity. Thus, the inventors have decided to further investigate this strain.

### A.2 Bacterial culture of MB9

Bacteria were grown in liquid BHIS medium (brain-heart infusion medium supplemented with 0.5% yeast extract [Difco]) supplemented with cellobiose (1 mg/ml [Sigma]), maltose (1 mg/ml [Sigma]), and cysteine (0.5 mg/ml [Sigma]) inside an anaerobic chamber (N2 = 90%, CO2 = 5% and H2 = 5%). The cryotubes with frozen bacterial cultures and 16% glycerol were defrosted and revived at a 10% inoculation in liquid BHIS for 24 hours at 37°C. Then 1% inoculations were made for overnight (O/N) growth (16-18 hours) at 37°C. The next morning, supernatants from 2 mL of the cultures were taken for SCFA analysis (A.4) and HT-29 cell co-incubation, after centrifugations of 2 times 10 minutes at 4500-5000g at 4°C outside of the anaerobic chamber. The supernatants were stored at -20°C until use.

Growth curves were measured by taking the OD600 every hour from a 2% culture, which was inoculated from the 1% overnight culture that was described above (Figure 1A). Every two hours dilutions were made and plated on BHIS agar plates for Colony Forming Unit (CFU) counting (Figure 1B). The plates were incubated at 37°C for 2 days. All materials were introduced into the anaerobic chamber at least 5 days prior the experiments in order to allow for deoxygenation.

BHIS composition is liquid BHIS medium (brain-heart infusion medium supplemented with 0.5% yeast extract [Difco]) supplemented with cellobiose (1 mg/ml [Sigma]), maltose (1 mg/ml [Sigma]), and cysteine (0.5 mg/ml [Sigma]).

### A32 Oxygen sensitivity determination

The isolated strains were tested for oxygen sensitivity by plating them from their respective 1% cultures. With 1 loop per strain the bacteria were individually plated on BHIS-agar plates, which were brought inside the anaerobic chamber one week on beforehand. The plates were then taken out of the anaerobic chamber into ambient atmosphere for 10, 20, 30, 40, 50 and 60 minutes and one plate per strain stayed inside the anaerobic chamber as a control. After the required time in ambient atmosphere, the plates were incubated at 37°C inside the anaerobic chamber. The maximum resisted oxygen concentration per strain was determined by whether at least 1 colony would grow at its respective time or not.

### A.4 Short chain fatty acid analysis

Short chain fatty acid analysis was performed by means of gas chromatography (AutoSystem XL, PerkinElmer and 7890B, Agilent Technologies). The SCFAs that could be detected were acetate, propionate, isobutyrate, butyrate, isovalerate, valerate, isocaproate and caproate. The collected bacterial supernatants were defrosted and an internal standard of 2-ethylbutyrate was added in a 1:4 ratio in order to normalize the samples in one run. The measurements of the production of SCFAs by the bacterial samples were normalized by the measurements of the SCFA level in the (BHIS) medium without bacteria of the same experiment (10⁸-10⁹ CFUs/mL after 11-17 hours of growth).

### A.5 Sequencing of the 16S rDNA

A 16S rDNA is sequenced by using the primers fDl (5'-ccgaattcgtcgacaacAGAGTTTGATCCTGGCTCAG-3' - SEQ ID NO. 2) and rp2 (5'-cccgggatccaagcttACGGCTACCTTGTTACGACTT-3' - SEQ ID NO. 3) (Weisburg *et al.* 1991 Weisburg, W. G., S. M. Barns, D. A. Pelletier, and D. J. Lane.1991. 16S ribosomal DNA amplification for phylogenetic study. J. Bacteriol.173:697-703) and Dreamtaq polymerase (Thermofisher) for amplification of DNA from a pure colony. Conditions were 95°C for 15 min, followed by 38 cycles of 95°C for 30 sec, annealing at 52°C for 30 sec, and extension at 72°C for 1,5 min, with a final extension at 72°C for 10 min for bacterial amplification and sequenced by Eurofins (Ebersberg).

### B. Results

### B.1. 16S rDNA of the MB9 strain

The 16S rDNA, corresponding to the 16S rRNA of the MB9 strain, has been sequenced by using the method described in the Materials and Methods.

The resulting 16S rDNA sequence consists of the sequence of SEQ ID NO. 1 below.

AGTCGAACGGAATATTTATTGAAACTTCGGT
GGATTTAATTTAGTTTCTAGTGGCGGACGGGTG
AGTAACGCGTGGGTAACCTGCCTTATACTGGGG
GATAACAGCCAGAAATGACTGCTAATACCGCATA
AGCGCACAGAACCGCATGGTTCGGTGTGAAAAA

CTCCGGTGGTATAAGATGGACCCGCGTTGGATT
AGCTGGTTGGCAGGGCAGCGGCCTACCAAGGC
GACGATCCATAGCCGGCCTGAGAGGGTGAACG
GCCACATTGGGACTGAGACACGGCCCAGACTC
CTACGGGAGGCAGCAGTGGGGAATATTGCACA
ATGGGGGAAACCCTGATGCAGCGACGCCGCGT
GAAGGAAGAAGTATCTCGGTATGTAAACTTCTAT
CAGCAGGGAAGATAATGACGGTACCTGACTAAG
AAGCCCCGGCTAACTACGTGCCAGCAGCCGCG
GTAATACGTAGGGGGCAAGCGTTATCCGGATTT
ACTGGGTGTAAAGGGAGCGTAGACGGCGCAGC
AAGTCTGATGTGAAAGGCAGGGGCTTAACCCCT
GGACTGCATTGGAAACTGCTGTGCTTGAGTGCC
GGAGGGGTAAGCGGAATTCCTAGTGTAGCGGTG
AAATGCGTAGATATTAGGAGGAACACCAGTGGC
GAAGGCGGCTTACTGGACGGTAACTGACGTTG
AGGCTCGAAAGCGTGGGGAGCAAACAGGATTA
GATACCCTGGTAGTCCACGCCGTAAACGATGAA
TACTAGGTGTCAGGGAGCACAGC (SEQ ID NO. 1)

Based on the 16S rDNA sequence of SEQ ID NO. 1, strain MB9 appears to be close to the commensal bacteria *Blautia faecis* and *Blautia luti.*

### B.2. Functional features of MB9 strain

B.2.1. MB9 strain may be classified in the group of bacteria that are hypersensitive to oxygen, since MB9 strain resists to oxygen during a maximum period of time of 10 minutes, as measured by the method discloses in the Materials end Methods.
B.2.2. MB9 stain consists of a commensal bacterium that overproduces acetate, since MB9 has been found producing 29.8 mmol/L acetate in the conditions discloses in the Materials and Methods (Interquartile Range (IQR)=9.5, n=6, after reaching early stationary phase or after an over night culture (O/N)).

Acetate production by a serial of commensal bacteria hypersensitive to oxygen originating from the same fecal sample, including MB9 strain, was assessed. The comparative results are presented in Figure 2. The results of Figure 2 show that among the 17 strains hypersensitive to oxygen which were tested, sole the MB9 strain overproduces acetate.
B.2.3. Growth of the MB9 strain in the BHIS (Brain Heart Infusion Supplemented with yeast extract 0.5% broth) culture medium has been measured. The results are depicted in figures 1A and 1B. The maximum OD of about 3.0 is reached in about 8-10 hours of growth with a maximum of 10⁹ CFUs/mL.
B.2.4. It has been ensured that the MB9 strain does not induce any pro-inflammatory response by using the test previously disclosed in Sokol et al., 2008 (Proc Natl Acad Sci U S A. 2008 Oct 28;105(43):16731-6. PMID: 18936492) and Kechaou et al., 2013 (Appl. Environ. Microbiol., 79(5), 1491-1499) PMID: 23263960). The existence of an induction of a pro-inflammatory response was thus assessed through measuring the TNF-alpha-stimulated IL-8 production in human HT-29 cells by means of Enzyme-linked immunosorbent assays (ELISA) with BioLegend's ELISA MAX™ Standard Sets for human IL-8. The results are shown in Figure 3. Neither in a TNFa-stimulated, nor in unstimulated conditions is the inhibition on IL-8 production in HT-29 cells different after the addition of supernatant of MB9, compared to the addition of only BHIS medium or only cell culture DMEM medium. Cells were stimulated for IL-8 production with TNFa and grown in DMEM medium. MB9 was grown in BHIS medium.

### Example 2 : Therapeutic effect of strain MB9 against bacterial superinfection

### A. Materials and Methods

### A.1 Infections, treatment with MB9 or F2 and assessment of bacterial loads (Figure 4)

For infection with IAV (Influenza A Virus), adult mice were anesthetized by intramuscular injection of 1.25 mg of ketamine plus 0.25 mg of xylazine in 100 µl of phosphate buffered saline (PBS), and then intranasally (i.n.) infected with 50 µl of PBS containing (or not, in a mock sample) 100 plaque forming units (p.f.u.) of the H1N1 A/California/04/2009 (pdm09) (Barthelemy *et al.,* 2017, 2018). This dose corresponds to a sub-lethal dose, which is necessary to investigate secondary bacterial infection.

For the probiotic treatment (Experiments Probio #1 to #6), mice were treated by gavage at 1 dpi (days post-infection) until 7 dpi by the probiotic MB9 or F2 in 200µL of BHIS medium. Vehicle mice received only 200µL of BHIS medium.

For secondary pneumococcal infection, IAV (H1N1, pdm2009)-infected mice were challenged at 8 dpi with a low dose (from 1,4x10³ c.f.u to 0.9 x10⁴ c.f.u) in 30µL of PBS intranasally of *S*. *pneumoniae* serotype 1, a serotype linked to invasive pneumococcal disease (clinical isolate E1586) was used. This dose is largely sufficient to allow bacterial outgrowth and dissemination. Bacteria in the lungs and spleen were counted 30 h after the *S. pneumoniae* challenge by plating serial 10-fold dilutions of lung or spleen homogenates onto blood agar plates. The plates were incubated at 37°C with 5% CO₂ overnight and viable bacteria were counted 24 h later. Survival and body weight were monitored daily after IAV infection and mice were euthanized when they lost in excess of 20% of their initial body weight.

Different doses of strains have been inoculated :
- Experiment #1 and #4: strain F2 : 2x10⁷ CFUs/200 µL per gavage per mouse daily; strain MB9 : 1.2x10⁵ CFUs/200 µL per gavage per mouse daily; *S. pneumoniae* : 1.4x10³ CFUs/30µL per intranasal per mouse.
- Experiment Probio#2: strain F2 : 4.2x10⁵ CFUs/200 µL per gavage per mouse daily; strain MB9 : 5.6x10⁶ CFUs/200 µL per gavage per mouse daily; *S. pneumoniae* : 2x10³ CFUs/30µL per intranasal per mouse.
- Experiment Probio#6 : strain F2 : 2.2x10⁷ CFUs/200 µL per gavage per mouse daily; strain MB9 : 6.8x10⁶ CFUs/200 µL per gavage per mouse daily; *S. pneumoniae* : 0.9x10⁴ CFUs/30µL per intranasal per mouse.

### B. Results

The MB9 strain was tested in a mouse model inoculated with the influenza virus (H1N1 virus) and undergoing a second infection with *Streptococcus pneumoniae* (see experimental protocol; figure 4). In an article submitted for publication, the co-inventors of the patent describe that the inoculation of the influenza virus causes a dysbiosis of the intestinal microbiota and a fall in the production of acetate by the intestinal microbiota. In addition, the addition of acetate to drinking water has a partial beneficial effect on superinfection with *Streptococcus pneumoniae.*

The survival of the tested mice after administering various doses of *S*. *pneumoniae* as a superinfection. The results are shown in figures 5A, 5B and 5C, respectively.

The mice are longer protected from death after secondary infection, when gavaged with MB9, both in the case of a superinfection of *S*. *pneumoniae* at a concentration of 2x10³ CFUs/30µL per intranasal per mouse and at 0.9x10⁴ CFUs/30µL per intranasal per mouse (Experiment #4: Vh n=8, F2 n=8, MB9 n=8, Experiment #6: Vh n=8, F2 n=8, MB9 n=8).

It has been further shown that strain MB9 reduces the weight loss that is observed during infection with the *Influenza* virus, as it is shown in figures 6A, 6B and 6C, respectively.

The results depicted in figures 6A-6C show that the weight of the *influenza* infected mice is less quickly affected when the mice are gavaged with MB9, before the superinfection of *S*. *pneumoniae.* (Experiment #2: Vh n=7, F2 n=6, MB9 n=8, Experiment #4: Vh n=8, F2 n=8, MB9 n=8, Experiment #6: Vh n=8, F2 n=8, MB9 n=8).
Still further, as it is shown in Figure 7, gavaging mice with strain MB9 reduces the pulmonary superinfection by *S. pneumoniae* during a primary infection with the *Influenza* virus. When gavaged with MB9, a significantly lower CFU count of the pathogen *S*. *pneumoniae* has been found in the lungs. (Experiment #1: Vh n=7, F2 n=10, MB9 n=9, Experiment #2: Vh n=6, F2 n=6, MB9 n=7).

Yet further, as it is shown in Figure 8, gavaging mice with strain MB9 reduces the systemic superinfection by *S. pneumoniae* during a primary infection with the *Influenza* virus. When gavaged with MB9, a lower CFU count of the pathogen *S. pneumoniae* has been found in the spleen. (Experiment #1: Vh n=7, F2 n=10, MB9 n=9, Experiment #2: Vh n=7, F2 n=6, MB9 n=8).

Also, the experimental results show that the protective effect of strain MB9 against a bacterial superinfection seems to be dose-dependent, as it is shown in Figures 9A, 9B, 9C and 9D, respectively.

Figure 9A represents the results of lung infection by *S. pneumoniae* (i) by administering BHIS medium at 200µL per gavage per mouse daily without administering any bacteria (Vh), (ii) by administering strain F2 at 2x10⁷ CFUs/200µL per gavage per mouse daily or (iii) by administering strain MB9 at 1.2x10⁵ CFUs/200 µL per gavage per mouse daily.

Figure 9B represents the results of systemic infection by *S. pneumoniae* (i) by administering BHIS medium at 200µL per gavage per mouse daily without administering any bacteria (Vh), (ii) by administering strain F2 at 2x10⁷ CFUs/200µL per gavage per mouse daily or (iii) by administering strain MB9 at 1.2x10⁵ CFUs/200 µL per gavage per mouse daily.

Figure 9C represents the results of lung infection by *S. pneumoniae* (i) by administering BHIS medium at 200µL per gavage per mouse daily without administering any bacteria (Vh), (ii) by administering strain F2 at 4.2x10⁵ CFUs/200µL per gavage per mouse or (iii) by administering strain MB9 at 5.6x10⁶ CFUs/200 µL per gavage per mouse daily.

Figure 9D represents the results of systemic infection by *S. pneumoniae* (i) by administering BHIS medium at 200µL per gavage per mouse daily without administering any bacteria (Vh), (ii) by administering strain F2 at 4.2x10⁵ CFUs/200µL per gavage per mouse daily or (iii) by administering strain MB9 at 5.6x10⁶ CFUs/200 µL per gavage per mouse daily.

The results presented in figures 9A-9D show that reduction of *S*. *pneumoniae* is similar for mice gavaged with MB9 in a concentration for both 1x10⁵ and 5x10⁶ CFUs/200µL per gavage per mouse daily. (Experiment #1: Vh n=7, F2 n=10, MB9 n=9, Experiment #2: Vh n=7, F2 n=6, MB9 n=8).

## Claims

1. A composition for use in a method for treating and/or preventing intestinal and/or pulmonary disease associated with gut dysbiosis in a subject in need thereof, wherein the composition comprises, in a physiologically acceptable condition, at least one acetate-overproducing bacteria hypersensitive to oxygen (AOHO bacteria).

2. The composition for its use according to claim 1, wherein the said at least one AOHO bacteria is **characterized by** a 16S rDNA having at least 90% nucleic acid identity with the nucleic acid sequence of SEQ ID NO. 1.

3. The composition for its use according to claim 1 or 2, wherein the AOHO bacteria in the composition are present in a range of 10³ to 10⁹ CFUs per dosage unit.

4. The composition for its use according to any of claims 1 to 3, wherein intestinal and/or pulmonary disease associated with gut dysbiosis is selected in the group comprising : cancer, periodontitis, inflammatory bowel disease, chronic fatigue syndrome, obesity, bacterial vaginosis, colitis, type II diabetes, allergies and frailty syndromes.

5. The composition for its use according to any of claims 1 to 4, wherein gut dysbiosis is a dysbiosis caused by impaired microbiota, antibiotic treatment, medicine, chemotherapies, alcohol, inappropriate diet, undernutrition, mal-nutrition, or virus.

6. The composition for its use according to any of claims 1 to 5, for achieving weight management in the subject.

7. The composition for its use according to any of claims 1 to 6, wherein the composition is formulated for oral or rectal administration.

8. The composition for its use according to any of claims 1 to 7, wherein the composition is in the form of a powder or granules, a food product, a beverage, a pharmaceutical, a nutraceutical, a food additive, a food supplement, a dairy product or a capsule.

9. A composition for use in a method for treating and/or preventing a bacterial superinfection associated with gut dysbiosis, preferably a bacterial pulmonary superinfection, in a subject in need thereof, wherein the composition comprises, in a physiologically acceptable condition, an effective amount of at least one AOHO bacteria.

10. A bacterial strain having one or more of the following features:
a. a 16S rDNA having at least 90% nucleic acid identity with the nucleic acid sequence of SEQ ID NO. 1,
b. it is hypersensitive to oxygen, and
c. it is an acetate-overproducing bacteria, and/or
d. it is an intestinal commensal bacteria.

11. A bacterial strain according to claim 10, wherein the bacterial strain produces at least 20mmol/L (mM) and at most 100 mM acetate.

12. A bacterial strain according to claim 10 or 11, for use in a method for treating and/or preventing intestinal and/or pulmonary disease in a subject in need thereof.

13. A bacterial strain according to claim 10 or 11, for use in a method for treating and/or preventing a bacterial superinfection associated with gut dysbiosis, preferably a bacterial pulmonary superinfection, in a subject in need thereof.


SEQUENCE LISTING

<110> Institut national de recherche pour l'agriculture, l'alimentation et
l'environnement (INRAE)
      Centre National de la Recherche Scientifique (CNRS)
      INSERM (Institut national de la santé et de la recherche
      médicale)
      Université de Lille
      Institut Pasteur de Lille

<120> Composition for treating intestinal or pulmonary diseases

<130> PR86087

<160> 3

<170> BiSSAP 1.3.2

<210> 1
<211> 775
<212> DNA
<213> Blautia

<223> "16S rDNA deposited in the name of Institut National de la Recherche
Agronomique (INRA) according to the Budapest Treaty at the Collection
DSMZ-German Collection of Microorganisms and Cell Cultures GmbH on December
19, 2019 under the ref num: DSM 33383"

<220>
<223> 16S rDNA deposited in the name of Institut National de la
      Recherche Agronomique (INRA) according to the Budapest Treaty at
      the Collection DSMZ-German Collection of Microorganisms and Cell
      Cultures GmbH on December 19, 2019 under the ref num: DSM 33383

<400> 1
agtcgaacgg gaatatttta ttgaaacttc ggtggattta atttagtttc tagtggcgga      60

cgggtgagta acgcgtgggt aacctgcctt atactggggg ataacagcca gaaatgactg     120

ctaataccgc ataagcgcac agaaccgcat ggttcggtgt gaaaaactcc ggtggtataa     180

gatggacccg cgttggatta gctggttggc agggcagcgg cctaccaagg cgacgatcca     240

tagccggcct gagagggtga acggccacat tgggactgag acacggccca gactcctacg     300

ggaggcagca gtggggaata ttgcacaatg ggggaaaccc tgatgcagcg acgccgcgtg     360

aaggaagaag tatctcggta tgtaaacttc tatcagcagg gaagataatg acggtacctg     420

actaagaagc cccggctaac tacgtgccag cagccgcggt aatacgtagg gggcaagcgt     480

tatccggatt tactgggtgt aaagggagcg tagacggcgc agcaagtctg atgtgaaagg     540

caggggctta acccctggac tgcattggaa actgctgtgc ttgagtgccg gaggggtaag     600

cggaattcct agtgtagcgg tgaaatgcgt agatattagg aggaacacca gtggcgaagg     660

cggcttactg gacggtaact gacgttgagg ctcgaaagcg tggggagcaa acaggattag     720

ataccctggt agtccacgcc gtaaacgatg aatactaggt gtcagggagc acagc          775


<210> 2

<211> 37
<212> DNA
<213> Artificial Sequence

<223> "primer fD1"

<220>
<223> primer fD1

<400> 2
ccgaattcgt cgacaacaga gtttgatcct ggctcag                                37


<210> 3
<211> 37
<212> DNA
<213> Artificial Sequence

<223> "primer rp2"

<220>
<223> primer rp2

<400> 3
cccgggatcc aagcttacgg ctaccttgtt acgactt                                37